# EUROPEAN PATENT APPLICATION

(11) **EP 4 483 817 A1**
(43) Date of publication of application: **01.01.2025**
(21) Application number: 23759287.8
(22) Date of filing: 24.02.2023
(51) Int. Cl.: A61B 17/12

(54) **OCCLUDER**

(30) Priority: 25.02.2022 CN 202210178730
(71) Applicant: SHANGHAI SHAPE MEMORY ALLOY CO., LTD, Shanghai, 201612 (CN)
(72) Inventor: CHEN, Juan, Shanghai 201612 (CN); HU, Jinpeng, Shanghai 201612 (CN); WANG, Yunbing, Shanghai 201612 (CN); WANG, Fan, Shanghai 201612 (CN); LIU, Can, Shanghai 201612 (CN); LIU, Dezhong, Shanghai 201612 (CN); QIN, Yongwen, Shanghai 201612 (CN); PAN, Xiangbin, Shanghai 201612 (CN)
(74) Representative: Canzler & Bergmeier Patentanwälte Partnerschaft mbB
(86) International application number: PCT/CN2023/078248
(87) International publication number: WO 2023/160665

(57) **Abstract**

Disclosed is an occluder, made of a degradable material and comprising a net-shaped framework and a locking member; the net-shaped framework comprises an upper disc surface, a waist part and a lower disc surface which are formed in sequence, the lower disc surface is provided with a melt tail end of the net-shaped framework, and a limiting through hole is formed in the melt tail end; the locking member comprises a tensioning part and a locking part, the tensioning part is radial, a diffusion end of the tensioning part is connected to the upper disc surface, a contraction end of the tensioning part is connected to the locking part, a locking ring is provided at the proximal end of the locking part, the diameter of the locking ring is greater than that of the distal end of the limiting through hole, and the locking ring can be slotted into/penetrate out of the limiting through hole in the melt tail end by means of deformation. The occluder provided by the present invention is not easy to fall off and has a good occlusion effect.

## Description

### FIELD

The present invention relates to the technical field of interventional medical devices, and in particular to an occluder.

### BACKGROUND

Common congenital heart diseases include several types such as ventricular septal defect, atrial septal defect, patent ductus arteriosus and patent foramen ovale. In the past, the only effective method to treat the congenital heart diseases was surgical thoracotomy. Nowadays, with the development of medical devices and surgical techniques, minimally invasive interventional therapy is gradually mature and replaces the surgical thoracotomy. The minimally invasive interventional therapy overcomes many defects of the thoracotomy such as much pain, great trauma, high cost and high risk, and further overcomes defects that recurrence easily happens due to simple injection and pulmonary embolism is easily caused (once the pulmonary embolism occurs, life is in danger).

For a conventional minimally invasive interventional surgery, a heart occluder is mainly adopted for occlusion treatment. Most of the mainstream occluders are made of a nickel-titanium alloy material in the current market, which achieves occlusion treatment of defect sites by utilizing super-elastic properties of the nickel-titanium alloy material. However, a conventional metal occluder will be accompanied by complications of a certain ratio during treatment. In addition, the occluder mainly acts as a bridge in a human body and facilitates endothelialization of surrounding tissues on the surface of the occluder, thereby achieving occlusion of the defects. However, the mission of the occluder has been completed after complete endothelialization, the occluder still remains in the body and will cause a series of complications including allergy and arrhythmia caused by the release of nickel ions. Since the nickel-titanium alloy material is non-degradable, the metal occluder will permanently exist in the heart after being implanted into the human body, thereby increasing occurrence probabilities of the complications.

Based on the above problems, some degradable occluders are present in the current market. However, since the elasticity of the degradable material is far lower than that of the nickel-titanium alloy material, the occluders are often easy to fall off in an occlusion operation so as to cause failure of the operation.

### SUMMARY

A purpose of embodiments of the present invention is to overcome defects in the prior art and provide an occluder. The occluder is difficult to fall off and excellent in occlusion effect. Specific technical solutions are as follows.

The embodiments of the present invention provide an occluder. The occluder is made of a degradable material and includes a net-shaped framework and a locking member.

The net-shaped framework includes an upper disc surface, a waist part and a lower disc surface which are formed in sequence, the lower disc surface is provided with a melt tail end of the net-shaped framework, and a limiting through hole is formed in the melt tail end.

The locking member includes a tensioning part and a locking part. The tensioning part is radial, a diffusion end of the tensioning part is connected to the upper disc surface, and a contraction end of the tensioning part is connected to the locking part. A locking ring is provided at the proximal end of the locking part, the diameter of the locking ring is greater than that of the distal end of the limiting through hole, and the locking ring can be slotted into/penetrate out of the limiting through hole in the melt tail end by means of deformation.

In some embodiments, the limiting through hole includes a plurality of first through holes in serial communication with one another, the opening diameter of the distal ends of the first through holes is smaller than the diameter of the locking ring, the opening diameter of the proximal ends of the first through holes is greater than the diameter of the locking ring, and preferably the longitudinal sections of the first through holes are trapezoidal.

In some embodiments, the limiting through hole further includes a second through hole in communication with the first through hole at the proximal end, and the opening diameter of the distal end of the second through hole is equal to the opening diameter of the proximal end of the second through hole.

In some embodiments, the locking part further includes a pull thread, the distal end of the pull thread is fixed on the upper disc surface, and the proximal end of the pull thread is wound to form the locking ring.

In some embodiments, the opening diameter of the distal end of the limiting through hole is smaller than the opening diameter of the proximal end of the limiting through hole, the locking ring includes a plurality of locking ring units connected in series, and the locking ring units can enter/penetrate out of the limiting through hole one by one from the opening at the distal end of the limiting through hole by means of deformation.

In some embodiments, the pull thread is made of a degradable material, and a developing thread is wound on the pull thread.

In some embodiments, an external thread is formed on the surface of the melt tail end, and the external thread is connected to an external push device.

In some embodiments, the side wall of the melt tail end is further provided with a developing ring arranged surrounding the limiting through hole.

In some embodiments, the occluder further includes flow blocking membranes, wherein the flow blocking membranes are made of a degradable material and includes a first flow blocking membrane covering an inner wall of the upper disc surface, a second flow blocking membrane located on the cross section of the waist part, and a third flow blocking membrane covering an inner wall of the lower disc surface.

In some embodiments, the tensioning part includes a plurality of groups of degradable pull belts, the proximal ends of each group of pull belts gather to be fixed on the locking part, and the distal ends of each group of pull belts penetrate through the third flow blocking membrane, the second flow blocking membrane and the first flow blocking membrane in sequence to be dispersedly fixed on the upper disc surface.

In some embodiments, each group of pull belts is composed of a plurality of degradable threads.

In some embodiments, the waist part of the net-shaped framework can penetrate through the septal defect of body tissues, and when the locking ring is limited in the limiting through hole of the melt tail end, the locking part drives the tensioning part to enable the upper disc surface to be close to the lower disc surface, so that the upper disc surface and the lower disc surface produce a clamping force around the septal defect to enable the occluder to occlude the septal defect.

In some embodiments, the locking ring can enter the limiting through hole by means of deformation and is slotted into the limiting through hole.

Beneficial effects brought by the technical solutions provided by the present invention are as follows.

With the adoption of the occluder, after the waist part of the net-shaped framework penetrates through the septal defect and the upper disc surface and the lower disc surface are respectively occluded on two sides of the septal defect, and on the basis that the distal end of the tensioning part of the locking member can be fixed on the upper disc surface in a radial manner, the proximal end gathers on the locking part of the locking member, and the locking ring of the locking part can deform, when tension is applied to the locking ring, the locking part will drive the tensioning part to enable the upper disc surface to be close to the lower disc surface, so that the upper disc surface and the lower disc surface produce a certain clamping force around the septal defect. After the locking ring deforms to enter the limiting through hole via the opening at the distal end of the limiting through hole, and on the basis that the diameter of the locking ring is greater than the diameter of the distal end of the limiting through hole, when the tension disappears, the locking ring will be limited in the limiting through hole in the melt tail end, thereby ensuring the occlusion effect of the present disclosure and solving a problem that the occluder is easy to fall off in the occlusion operation due to insufficient elasticity of the material.

### BRIEF DESCRIPTION OF THE DRAWINGS

To clearly describe the technical solutions in the embodiments of the present invention, drawings required to be used in descriptions of the embodiments will be briefly introduced below. Apparently, the drawings described below are merely some embodiments of the present invention. Other drawings may also be obtained by those ordinarily skilled in the art according to the provided drawings without making creative labor.
Fig. 1 is a stereogram of an occluder provided by one embodiment of the present invention;
Fig. 2 is a front view of an occluder provided by one embodiment of the present invention;
Fig. 3 is a top view of an occluder provided by one embodiment of the present invention;
Fig. 4 is a section view of a net-shaped framework along a line A-A in Fig. 2;
Fig. 5 is a schematic structural diagram of a locking member provided by one embodiment of the present invention;
Fig. 6 is a section view of a melt tail end along a line A-A in Fig. 2;
Fig. 7 is a schematic diagram of the connection between a locking member and a melt tail end in one embodiment of the present invention;
Fig. 8 is a section view of a melt tail end along a line A-A in Fig. 2 in another embodiment of the present invention;
Fig. 9 is a section view of an occluder, taken along line A-A in Fig. 2, after it has been implanted into the body in another embodiment of the present invention;
Fig. 10 is a schematic structural diagram of a locking part in another embodiment of the present invention; and
Fig. 11 is a section view of an occluder, taken along line A-A in Fig. 2, after it has been implanted into the body in another embodiment of the present invention.

Reference symbols:
net-shaped framework-100; upper disc surface-110; waist part-120; lower disc surface-130; melt tail end-140; limiting through hole-141; first through hole-1411; second through hole-1412; developing ring-142; external thread-143;
first flow blocking membrane-210; second flow blocking membrane-220; third flow blocking membrane-230;
locking member-300; tensioning part-310; pull belt-311; locking part-320; pull thread-321; locking ring-322; locking unit-3221;
septal defect-400.

### DETAILED DESCRIPTION

To make purposes, technical solutions and advantages of the embodiments of the present invention more clear, the technical solutions in the embodiments of the present invention will be clearly and fully described below. It should be noted that, terms mentioned hereinafter are defined by considering functions in the present invention, and may be different according to the intentions or conventions of users and operators. Therefore, the terms should be defined based on the overall content of the present specification.

For example, direction or position relations indicated by terms such as "center", "upper", "lower", "left", "right", "vertical", "horizontal", "inner" and "outer" are direction or position relations shown based on the drawings, and are merely for conveniently describing the present invention and simplifying the descriptions, rather than indicating or implying that the device or component must have a specific direction or must be configured or operated in a specific direction. Therefore, the terms cannot be understood as a limitation to the present invention. In addition, terms such as "first", "second" and "third" are merely for describing, but cannot be understood to indicate or imply relative importance.

In the descriptions of the present invention, it should be noted that, unless expressly stated and defined, terms such as "mount", "connected" and "connect" should have generalized understandings, for instance, being fixed connection, detachable connection or integrated connection; or mechanical connection or electrical connection; or direct connection, or connection through an intermediate, or internal communication of two components. Specific meanings of the above terms in the present invention can be understood by those ordinary skilled in the art according to specific circumstances.

In addition, as long as there is no conflict, technical features involved in different implementation modes of the present invention below can be in combination with one another.

An occluder made of an alloy material will cause risk of complications during the operation or while remaining in the patient's body after the operation. In related technologies, the occluder is made of a degradable material to solve the problems, but elasticity of the degradable material is lower than that of the alloy material, and the occluder is often easy to fall off in the occlusion operation so as to cause failure of the operation.

The present application is provided based on analysis and discovery of the above problems.

The present application provides an occluder. The occluder is made of a degradable material and includes a net-shaped framework and a locking member.

The net-shaped framework includes an upper disc surface, a waist part and a lower disc surface which are formed in sequence, the lower disc surface is provided with a melt tail end of the net-shaped framework, and a limiting through hole is formed in the melt tail end.

The locking member includes a tensioning part and a locking part. The tensioning part is radial, a diffusion end of the tensioning part is connected to the upper disc surface, and a contraction end of the tensioning part is connected to the locking part. A locking ring is provided at the proximal end of the locking part, the diameter of the locking ring is greater than that of the distal end of the limiting through hole, and the locking ring can be slotted into/penetrate out of the limiting through hole in the melt tail end by means of deformation.

With the adoption of the occluder, after the waist part of the net-shaped framework penetrates through the septal defect and the upper disc surface and the lower disc surface are respectively occluded on two sides of the septal defect, and on the basis that the distal end of the tensioning part of the locking member can be fixed on the upper disc surface in a radial manner, the proximal end gathers on the locking part of the locking member, and the locking ring of the locking part can deform, when tension is applied to the locking ring, the locking part will drive the tensioning part to enable the upper disc surface to be close to the lower disc surface, so that the upper disc surface and the lower disc surface produce a certain clamping force around the septal defect. After the locking ring deforms to enter the limiting through hole via the opening at the distal end of the limiting through hole, and on the basis that the diameter of the locking ring is greater than the diameter of the distal end of the limiting through hole, when the tension disappears, the locking ring will be limited in the limiting through hole in the melt tail end, thereby ensuring the occlusion effect of the present disclosure and solving a problem that the occluder is easy to fall off in the occlusion operation due to insufficient elasticity of the material.

To clearly understand the occluder provided by the embodiments of the present disclosure, specific embodiments of the present invention will be described below with reference to the drawings. In the process, thicknesses of lines shown in the drawings or sizes of structural elements may be illustrated in an exaggerated way for specificity and convenience of the description.

### Embodiment 1

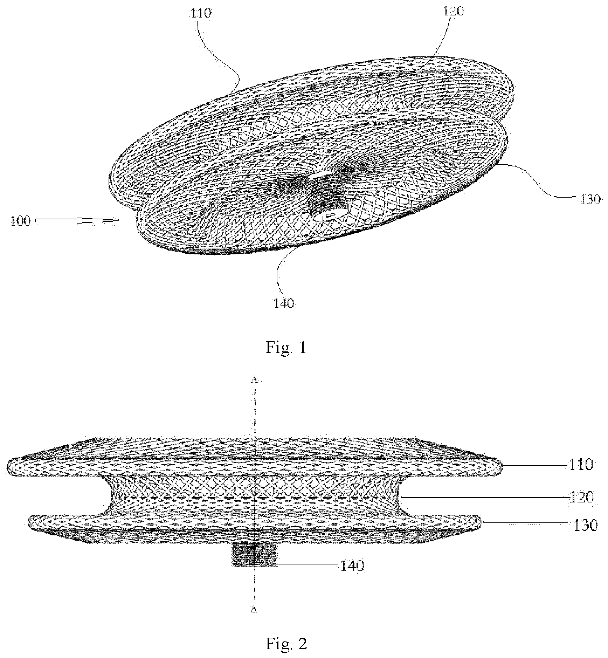

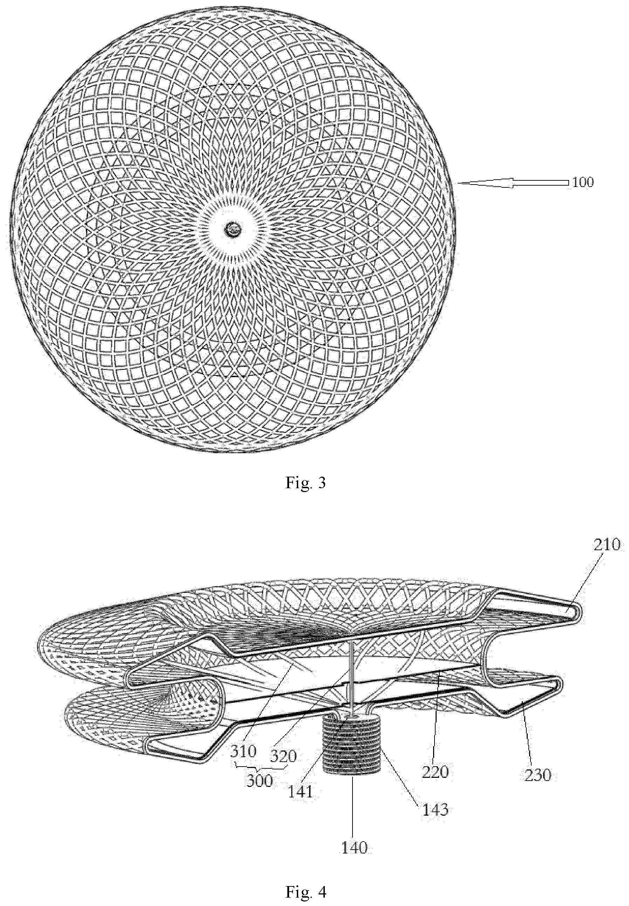

Referring to Figs. 1-6, in the present disclosure, the proximal end is one end close to an operator, and the distal end is one end away from the operator. Illustratively, the upper disc surface is one end away from the operator, and the melt tail end is one end close to an operator. The present application provides an occluder. Referring to Fig. 2, the occluder includes a net-shaped framework 100, flow blocking membranes and a locking member 300. The net-shaped framework 100 is made of a degradable material, illustratively, polylactic acid, polyglycolic acid, poly(p-dioxanone) or polyhydroxybutyric acid. The net-shaped framework 100 is generally formed by melting a tubular net structure with a necking port. Referring to Fig. 4, the net-shaped framework includes an upper disc surface 110, a waist part 120 and a lower disc surface 130 which are formed in sequence from the distal end to the proximal end, the necking port is located on the lower disc surface 130 and is melted to form a melt tail end 140, and a limiting through hole 141 is formed in the melt tail end 140. Referring to Figs. 1-3, the diameter of the upper disc surface 110 is 6-18 mm greater than the diameter of the waist part 120, the diameter of the lower disc surface 130 is 4-12 mm greater than the diameter of the waist part 120, and the diameter of the upper disc surface 110 is greater than the diameter of the lower disc surface 130. An external thread is formed on the surface of the melt tail end 140 to increase the surface roughness, thereby increasing a frictional force with an external push device and further ensuring delivery safety. The side wall of the melt tail end 140 is further provided with a developing ring 142 arranged around the limiting through hole 141.

Referring to Fig. 4 again, the flow blocking membranes are made of a degradable material, and include a first flow blocking membrane 210 covering the inner wall of the upper disc surface 110, a second flow blocking membrane 220 located on the cross section of the waist part 120, and a third flow blocking membrane 230 covering the inner wall of the lower disc surface 130. The first flow blocking membrane 210, the second flow blocking membrane 220 and the third flow blocking membrane 230 are sewed with the net-shaped framework 100 together through degradable threads. Materials of the first flow blocking membrane 210, the second flow blocking membrane 220, the third flow blocking membrane 230 and the degradable threads may include amorphous polylactic acid, L-polylactic acid, D-polylactic acid, poly(p-dioxanone), polycaprolactone, polyglycolic acid or poly(lactic-co-glycolic acid) copolymer, in the present embodiment, preferably poly(p-dioxanone), polycaprolactone, polyglycolic acid, poly(lactic-co-glycolic acid) copolymer, or any one or a blend of amorphous polylactic acid, L-polylactic acid and D-polylactic acid. The structure of the flow blocking membranes is spunlace non-woven fabric, needled non-woven fabric, spunbond non-woven fabric, thermal bonded non-woven fabric, wet non-woven fabric or electrospinning fiber membrane. The number of layers of the flow blocking membranes may be 2-6.

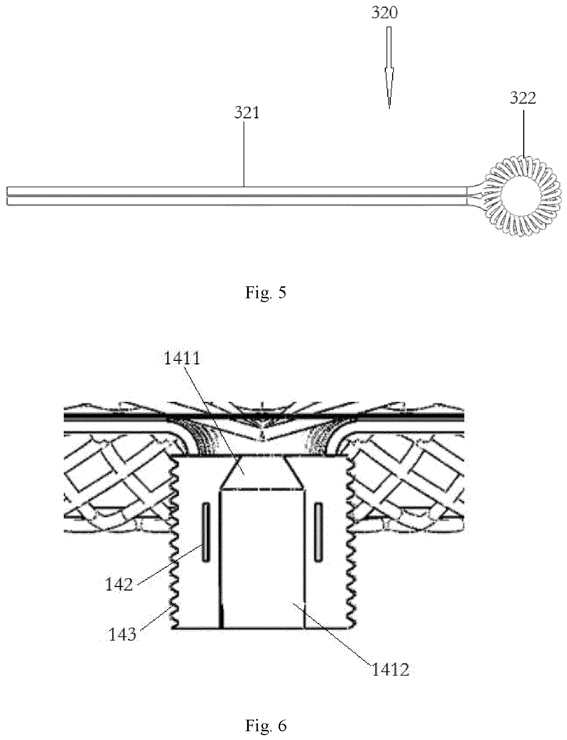

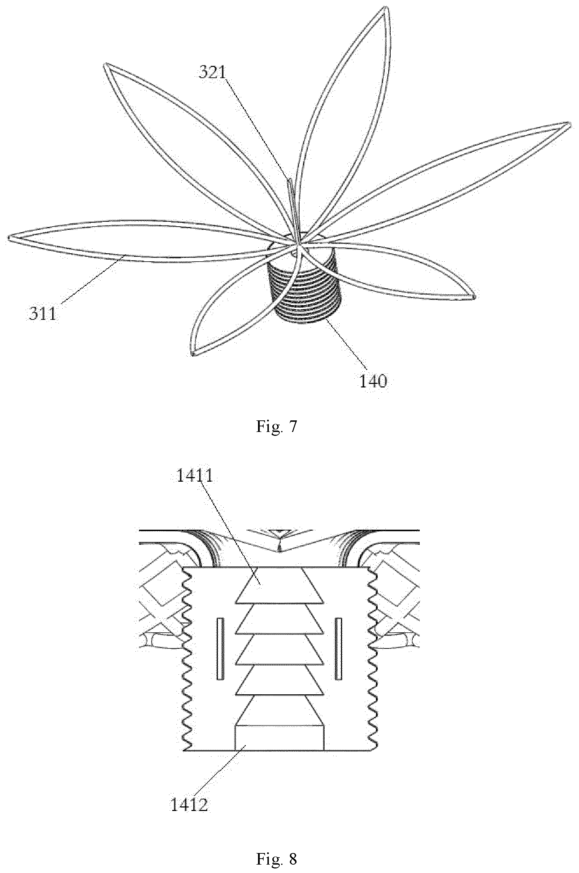

Referring to Figs. 4 and 7, the locking member 300 includes a tensioning part 310 and a locking part 320. The tensioning part 310 is radial, the tensioning part 310 includes a plurality of groups of degradable pull belts 311, the proximal ends, namely contraction ends, of each group of pull belts 311 gather to be fixed on the locking part 320, and the distal ends, namely diffusion ends, of each group of pull belts 311 penetrate through the third flow blocking membrane 230, the second flow blocking membrane 220 and the first flow blocking membrane 210 in sequence to be dispersedly fixed on the upper disc surface 110. Each group of pull belts 311 is composed of a plurality of degradable threads. The proximal ends of the plurality of degradable threads in each group of pull belts 311 gather to be connected, and the distal ends of the plurality of degradable threads in each group of pull belts 311 gather to be connected. In the present embodiment, each group of pull belts 311 is composed of two degradable threads. Referring to Figs. 4 and 5, the locking part 320 is composed of a pull thread 321 and a locking ring 322, the distal end of the pull thread 321 penetrates through the third flow blocking membrane 230, the second flow blocking membrane 220 and the first flow blocking membrane 210 in sequence to be fixed on the upper disc surface 110, the proximal end of the pull thread 321 is wound to form the locking ring 322, and developing threads are wound on the pull thread 321 and the locking ring 322 of the locking part 320. Referring to Figs. 4 and 6, the diameter of the locking ring 322 is greater than that of the distal end of the limiting through hole 141, and the locking ring 322 can be slotted into/penetrate out of the limiting through hole 141 in the melt tail end 140 by means of deformation.

With the adoption of the occluder, after the waist part 120 of the net-shaped framework 100 penetrates through the septal defect 400 and the upper disc surface 110 and the lower disc surface 130 are respectively occluded on two sides of the septal defect 400, and on the basis that the distal end of tensioning part 310 of the locking member 300 can be fixed on the upper disc surface 110 in a radial manner, the proximal end gathers on the locking part 320 of the locking member 300, and the locking ring 322 of the locking part 320 can deform, when tension is applied to the locking ring 322, the locking part 320 will drive the tensioning part 310 to enable the upper disc surface 110 to be close to the lower disc surface 130, so that the upper disc surface 110 and the lower disc surface 130 produce a certain clamping force around the septal defect 400. After the locking ring 322 deforms to enter the limiting through hole 141 via the opening at the distal end of the limiting through hole 141, and on the basis that the diameter of the locking ring 322 is greater than the diameter of the distal end of the limiting through hole 141, when the tension disappears, the locking ring 322 will be limited in the limiting through hole 141 in the melt tail end 140, thereby ensuring the occlusion effect of the present disclosure and solving a problem that the occluder is easy to fall off in the occlusion operation due to insufficient elasticity of the material. In addition, the developing threads are wound on the pull thread 321 and the locking ring 322 of the locking part 320, and the developing ring 142 is embedded into the melt tail end 140. Therefore, in the surgical imaging process, when the locking ring 322 is pulled, the locking ring 322 will pass the developing ring 142, so that whether the occluder is locked can be clearly judged to realize visual operations, thereby significantly increasing efficiency of the operation.

In the related technologies, the occluder is applicable to a certain kind of operation environments only, but cannot be well occluded at the lesions in different types of operation environments. To further increase the universality and occlusion effect of the present disclosure, technical solutions of the present application are provided below.

### Embodiment 2

Referring to Fig. 5, in the present embodiment, the locking ring 322 is a spring coil wound by the degradable pull thread 321, developing threads are wound on the pull thread 321 and the locking ring 322, and the locking ring 322 is provided with a center circle, such that the locking ring 322 has an elastic deformation space.

Referring to Fig. 8, a forming process of the melt tail end 140 is as follows: the degradable threads are subjected to hot melt forming to form a whole with the net-shaped framework 100, wherein the limiting through hole 141 includes a plurality of first through holes 1411 in serial communication with one another, the opening diameter of the distal ends of the first through holes 1411 is smaller than the diameter of the locking ring 322, the opening diameter of the proximal ends of the first through holes 1411 is greater than the diameter of the locking ring 322, for example, the longitudinal sections of the first through holes 1411 can be trapezoidal, and the limiting through hole 141 is a through hole formed by connecting N trapezoidal small holes.

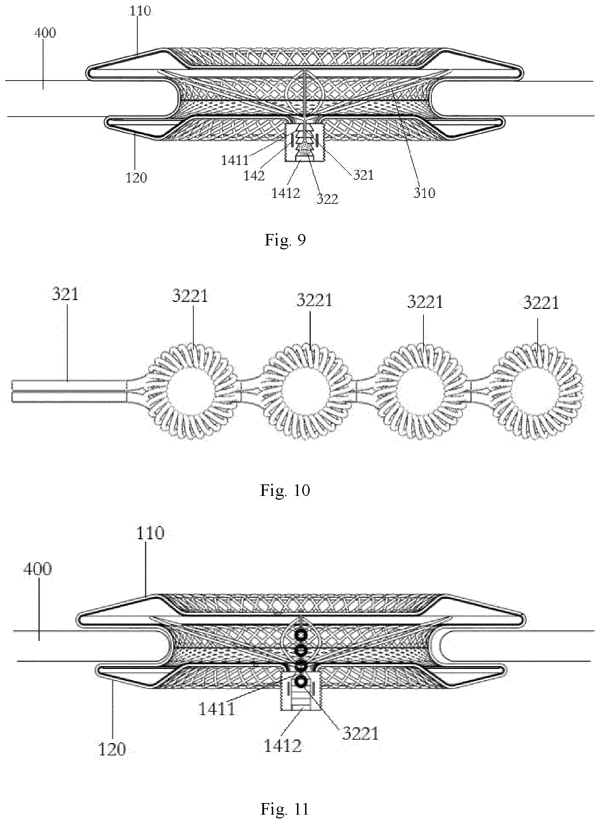

Fig. 9 is a section view of an occluder, taken along line A-A in Fig. 2, after it has been implanted into the body in the present embodiment, wherein 400 is the septal defect of cardiac tissues. After the occluder is released from the sheathing canal, by pulling the locking ring 322, since the outer diameter of the locking ring 322 is greater than the opening diameter of the distal ends of the first through holes 1411 and is smaller than the opening diameter of the proximal ends of the first through holes 1411, based on the structure of the center circle on the locking ring 322, the locking ring 322 can pass through the first through holes 1411 in presence of a certain tension, such that the upper disc surface 110 and the lower disc surface 130 produce a certain clamping force around the septal defect 400. When the locking ring 322 is pulled into different first through holes 1411, the pitch of the pull thread 321 will change, so that the tensioning part 310 is driven to decrease the distance between the upper disc surface 110 and the lower disc surface 130. Thus, the clamping force around the septal defect 400 is increased, such that the occluder may be locked at different positions for the septal defect 400 of different thicknesses, thereby achieving more effective occlusion.

In addition, the limiting through hole 141 further includes a second through hole 1412 in communication with the first through hole 1411 at the proximal end, and the opening diameter of the distal end of the second through hole 1412 is equal to the opening diameter of the proximal end of the second through hole 1412. It is generally understood that, the second through hole 1412 is a cylindrical hole of which the diameter is equal to the diameter of the distal end of the first through hole 1411. According to the arrangement, since the diameter of the second through hole 1412 is greater than the maximum outer diameter of the locking ring 322, the locking ring 322 can be limited in the melt tail end 140 in a final locking state, thereby facilitating later endothelialization of the occluder.

### Embodiment 3

Referring to Figs. 10-11, in the present embodiment, the differences from embodiment 2 are as follows: the opening diameter of the distal end of the limiting through hole 141 is smaller than the opening diameter of the proximal end of the limiting through hole 141, for example a trapezoidal hole, the locking ring 322 includes a plurality of locking ring units 3221 connected in series, and the locking ring units 3221 can enter/penetrate out of the limiting through hole 141 one by one from the opening in the distal end of the limiting through hole 141 by means of deformation. Certainly, the limiting through hole 141 may include first through holes 1411 and a second through hole 1412 connected in series, wherein the first through hole 1411 is a trapezoidal hole, the second through hole 1412 is a cylindrical hole, and the diameter of the second through hole 1412 is greater than the maximum outer diameter of the locking ring units 3221. Therefore, the locking ring units 3221 can be limited in the melt tail end 140 in a final locking state, thereby facilitating later endothelialization of the occluder.

It should be noted that, the septal defect mainly refers to atrial septal defect or ventricular septal defect. The atrial septal defect is as follows: an abnormal channel exists between the left and right atria, and the normal anatomic structure is destroyed, such that blood can reach the opposite atrium from the ischemic location. The ventricular septal defect is as follows: an abnormal channel exists between the left and right atria, the normal anatomic structure is destroyed, and due to a higher pressure between the ventricles on two sides, the blood can flow from one side to the other side. The blood mainly shunts from the left side to the right side early, and later, the blood may be in a right-to-left shunt state, a left-to-right shunt state and a bidirectional shunt state. Body tissues are tissues that constitute the organism.

The above descriptions are merely preferred embodiments of the present invention and cannot be used for limiting the protection scope of the present invention. Modifications, equivalent replacements and improvements made in the spirit and principles of the present invention shall fall within the protection scope of the present invention.

## Claims

1. An occluder, made of a degradable material and comprising a net-shaped framework and a locking member, wherein
the net-shaped framework comprises an upper disc surface, a waist part and a lower disc surface which are formed in sequence, the lower disc surface is provided with a melt tail end of the net-shaped framework, and a limiting through hole is formed in the melt tail end; and
the locking member comprises a tensioning part and a locking part, wherein the tensioning part is radial, a diffusion end of the tensioning part is connected to the upper disc surface, and a contraction end of the tensioning part is connected to the locking part; a locking ring is provided at the proximal end of the locking part, the diameter of the locking ring is greater than that of the distal end of the limiting through hole, and the locking ring can be slotted into/penetrate out of the limiting through hole in the melt tail end by means of deformation.

2. The occluder according to claim 1, wherein the limiting through hole comprises a plurality of first through holes in serial communication with one another, the opening diameter of the distal ends of the first through holes is smaller than the diameter of the locking ring, and the opening diameter of the proximal ends of the first through holes is greater than the diameter of the locking ring.

3. The occluder according to any of the preceding claims, wherein longitudinal sections of the first through holes are trapezoidal.

4. The occluder according to any of the preceding claims, wherein the limiting through hole further comprises a second through hole in communication with the first through hole at the proximal end, and the opening diameter of the distal end of the second through hole is equal to the opening diameter of the proximal end of the second through hole.

5. The occluder according to any of the preceding claims, wherein the locking part further comprises a pull thread, the distal end of the pull thread is fixed on the upper disc surface, and the proximal end of the pull thread is wound to form the locking ring.

6. The occluder according to any of the preceding claims, wherein the opening diameter of the distal end of the limiting through hole is smaller than the opening diameter of the proximal end of the limiting through hole, the locking ring comprises a plurality of locking ring units connected in series, and the locking ring units can enter/penetrate out of the limiting through hole one by one from the opening at the distal end of the limiting through hole by means of deformation.

7. The occluder according to any of the preceding claims, wherein the pull thread is made of a degradable material, and a developing thread is wound on the pull thread.

8. The occluder according to any of the preceding claims, wherein an external thread is formed on the surface of the melt tail end, and the occluder is connected to an external push device through the external thread.

9. The occluder according to any of the preceding claims, wherein the side wall of the melt tail end is further provided with a developing ring arranged surrounding the limiting through hole.

10. The occluder according to any of the preceding claims, further comprising flow blocking membranes, wherein the flow blocking membranes are made of a degradable material and comprise a first flow blocking membrane covering an inner wall of the upper disc surface, a second flow blocking membrane located on the cross section of the waist part, and a third flow blocking membrane covering an inner wall of the lower disc surface.

11. The occluder according to any of the preceding claims, wherein the tensioning part comprises a plurality of groups of degradable pull belts, the proximal ends of each group of pull belts gather to be fixed on the locking part, and the distal ends of each group of pull belts penetrate through the third flow blocking membrane, the second flow blocking membrane and the first flow blocking membrane in sequence to be dispersedly fixed on the upper disc surface.

12. The occluder according to any of the preceding claims, wherein each group of pull belts is composed of a plurality of degradable threads.

13. The occluder according to any of the preceding claims, wherein the waist part of the net-shaped framework can penetrate through the septal defect of body tissues, and when the locking ring is limited in the limiting through hole of the melt tail end, the locking part drives the tensioning part to enable the upper disc surface to be close to the lower disc surface, so that the upper disc surface and the lower disc surface produce a clamping force around the septal defect to enable the occluder to occlude the septal defect.

14. The occluder according to any of the preceding claims, wherein the locking ring can enter the limiting through hole and is slotted into the limiting through hole by means of deformation.
